# EUROPEAN PATENT SPECIFICATION

(11) **EP 1 033 939 B1**
(45) Date of publication and mention of the grant of the patent: **19.01.2005**
(21) Application number: 98962393.9
(22) Date of filing: 25.11.1998
(51) Int. Cl.: A61B 17/068

(54) **DEVICE FOR INSTALLING A TISSUE FASTENER**
VORRICHTUNG ZUM ANBRINGEN CHIRURGISCHER KLAMMERN
DISPOSITIF SERVANT A PLACER UN ELEMENT DE FIXATION DANS DES TISSUS

(30) Priority: 26.11.1997 US 979872; 17.02.1998 US 24359
(43) Date of publication of application: 13.09.2000
(73) Proprietor: Linvatec Biomaterials Oy, 33720 Tampere (FI)
(72) Inventor: TORMALA, Pertti, FIN-33720 Tampere (FI); KARHI, Olli, FIN-90650 Oulu (FI); TAMMINMAKI, Markku, FIN-33400 Tampere (FI)
(74) Representative: KEIL & SCHAAFHAUSEN Patentanwälte
(86) International application number: PCT/EP1998/007585
(87) International publication number: WO 1999/026544

(56) References cited:
- EP-A- 0 463 551
- EP-A- 0 598 462
- EP-A- 0 770 354
- DE-A- 4 303 544
- US-A- 4 540 110
- US-A- 4 595 007
- US-A- 4 929 239
- US-A- 5 562 682
- US-A- 5 569 264

## Description

### Field of the Invention

This invention relates to the field of surgical devices. More specifically, this invention relates to an improved surgical device for inserting tissue fasteners.

The surgical device of the invention is particularly but not solely intended to be used in repair surgery of traumas of soft and/or tough tissues containing fibrous structures, such as knee meniscal tissues.

### Background of the Invention

In the past, doctors have effectively treated internal ruptures and tears of tissue by suturing, often with bioabsorbable sutures. For instance, this technique for treating ruptures of meniscal tissue in the knee has been described in N.A. Palmeri, T.F. Winters, A. E. Joiner and T. Evans, "The Development and Testing of the Arthroscopic Meniscal Staple", Arthroscopy, Vol. 5, No. 2, 1989, p. 156. However, suturing, particularly arthroscopic suturing, has many drawbacks. It is a complicated and tedious technique where risks for the patient are significant because of the danger to vessels and nerves. Additionally, the suturing of a ruptured meniscus leaves a single or several loops of sutures on the surface of the meniscal tissue, which can irritate joint cavity tissues. Therefore, for a long time surgeons have desired an absorbable fixation device, like a staple or fastener, which has the advantages of absorbable techniques but which can be used more rapidly and safely than sutures.

Tissue fasteners have been developed, including fasteners that may be inserted entirely below the surface of the tissue that is being treated, thereby preventing any irritation that may result from the portion of the fastener remaining above the ruptured tissue surface. These fasteners are described in detail in WO 99/01071, entitled SURGICAL FASTENER FOR TISSUE TREATMENT, by Tormala, et al.

Accordingly, there is a need for surgical devices to insert these fasteners. Such devices must be accurate, reliable, quick, easily positioned and operated within a patient, and cost effective. It is important to reduce the invasiveness and length of any surgery to repair internal ruptured tissues.

Certain previous devices for installing tissue fasteners require that the fasteners be manually inserted into the patient. It is time consuming for fasteners to be inserted with such devices because the surgeon has to, for instance, repeatedly tap the fastener until it is fully inserted into the patient. Further, because of the manual propulsion of the fastener, it is impossible with such devices to ensure that each fastener receives a measured, consistent amount of force to drive it into the patient.

Other previous devices for installing tissue fasteners have used mechanical techniques for propelling fasteners into a patient, but have only had the capability of holding one fastener at a time, thereby requiring the surgeon repeatedly during an operation to remove the device from the patient, load another fastener, and reinsert the device into the patient. It is important that a surgeon be able to insert these fasteners precisely. Thus, it is time consuming to remove the device from the patient and then reposition it so that it is in position to deliver a fastener exactly where needed. Having to do so increases the length and difficulty of the surgery, and the concomitant risk of infection or other complications to the patient.

There have also been devices for installing tissue fasteners that are capable of holding a fixed number of fasteners. These devices, however, are self contained and are not capable of receiving additional fasteners. These devices also do not provide enough flexibility to the surgeon concerning the number of fasteners used during the operation because in many cases, the surgeon will not know how many fasteners are needed until the operation has begun. For instance, if the device holds six fasteners, yet the operation demands eight fasteners, after the sixth fastener, the surgeon must remove the device, dispose of it, and insert a second new device containing another six fasteners into the patient. This device must then be disposed of after only two of its six fasteners have been inserted into the patient. The use of such a device increases the length and difficulty of the surgery, because the surgeon must change devices in the middle of the operation. This poses an added risk, because the second device could have slightly different operating characteristics than the first device to which the surgeon has become accustomed. Further, such devices are inefficient and costly because a single surgery could require the use of more than one delivery device. This is compounded by the fact that fasteners in the device that are not used are disposed of along with the delivery device.

For instance, United States Patent No. 5,569,264 describes a device for inserting implants into a patient that can hold more than one implant in a cassette or box. The box sits on top of the frame of the device and pushes implants from the box into the frame and into the channel through which the implants are inserted. The magazine must be filled with a suitable number of implants in advance of the operation. However, the surgeon will not always know how many implants are needed for a particular operation.

Further, none of these devices provide a method for inserting fasteners in such a way that no part of the fastener remains on or above the surface of the tissue being treated. It is advantageous to be able to insert fasteners entirely below the surface of the tissue being treated to avoid any irritation or inflammation that could occur when other tissues rub against that portion of the fastener on or above the tissue that has been treated.

An additional problem with previous devices in this field is that their conduits are invariably straight or invariably curved. At times, a surgeon is required to use these devices to repair tissue that is difficult to reach. Further, a surgeon must precisely insert many different fasteners at different angles. Thus, at times, a straight conduit may be preferred, while at other times, it may be difficult for the surgeon to properly approach the tissue that is to be treated with a straight conduit and a curved conduit may be preferred. Presently, in such a situation, a surgeon would need to use entirely separate devices with different conduits to insert the different fasteners. Using entirely different devices during an operation poses the same risk as described previously - the second device could have slightly different operating characteristics than the first device to which the surgeon has become accustomed. This increases the risk that a fastener will not be optimally inserted. Further, such devices are inefficient and costly because a single surgery could require the use of more than one delivery device.

Thus, there is a desire in the field for a device to install tissue fasteners that is capable of holding more than one fastener and capable of receiving additional fasteners during an operation without requiring the surgeon to remove the device from the patient. With such a device, the surgeon may use as many fasteners as he requires, while only having to insert the device into the patient once. Also, no fasteners are wasted. This reduces the length, difficulty, and cost of the procedure. Further, there is a desire in the field for a device that is capable of inserting a fastener entirely within the tissue to be treated so that no part of the fastener remains above or on the surface of the tissue. Such a device reduces the likelihood of irritation and inflammation of the treated area. Also, there is a desire in the field for a device that accurately and reliably inserts fasteners. Lastly, there is a desire in the field for a device with a conduit that can be curved or straight, to allow the surgeon to more easily insert the fastener properly into the patient.

Thus, it is an object of the present invention to provide a device for installing tissue fasteners that allows a minimally invasive method for repairing torn or ruptured tissue.

It is further an object of the present invention to provide a device for installing tissue fasteners that is capable of holding more than one fastener and of receiving additional fasteners during an operation without requiring the removal of the device from the patient.

It is further an object of the present invention to provide a device for installing tissue fasteners that is capable of inserting a fastener entirely within the tissue being treated, so that no part of the fastener remains above or on the surface of the treated tissue.

It is further an object of the present invention to provide a device for installing tissue fasteners that may quickly and easily be positioned within a patient.

It is further an object of the present invention to provide a device for installing tissue fasteners that accurately and reliably inserts tissue fasteners into a patient.

It is further an object of the present invention to provide a device for installing tissue fasteners that is cost effective.

These objects and others are attained with the device of the present invention, as described below.

### Summary of the Invention

The invention is defined in independent claim 1.

The device of the present invention is designed for repairing a tear in soft and/or tough tissue of a patient, such as a tear in the meniscus of the knee.

The device has a conduit, such as a cannula, that may be easily inserted into the patient and through which the fastener is delivered to the patient. This conduit is aligned with a seat for holding a fastener and a means for pushing a fastener, such as a piston, so that the pushing means is capable of pushing a fastener from its seat, through the conduit and into the patient. In a preferred embodiment of the invention, the shape of the conduit exactly matches the shape of the cross-section of the fastener so that the surgeon may more accurately direct the angle and location at which the fastener enters the patient. In another preferred embodiment, the pushing means may be made to slowly push the fastener from its seat and through the conduit until the distal end of the fastener contacts the surface of the tissue to be treated at the end of the conduit. At that time, the pushing means may be made to accelerate rapidly, thereby inserting the fastener into the tissue being treated. An advantage of this embodiment is that the fastener is less likely to become jammed in the conduit while being pushed slowly through it. Further, the conduit, piston, and fastener are subject to less wear, which helps to ensure proper functioning of the device during an operation.

The seat for holding fasteners is capable of holding a magazine containing one or more fasteners. When inserted into the seat, the magazine may be positioned so that a fastener is aligned with the pushing means and the conduit leading to the patient. Once a fastener has been inserted into the patient, the magazine may be manually positioned so that another fastener is shifted into position to be inserted. In one embodiment of this invention, the magazine may have means, such as a spring, for automatically moving a fastener into position for insertion once a fastener has been inserted.

The magazine may be easily removed from the seat during an operation, so that it may be replaced with a magazine containing one or more fasteners without requiring the conduit to be removed from the patient. Also, the same magazine can be removed, refilled with one or more additional fasteners, and reinserted into the seat, without requiring the removal of the conduit from the patient. In yet another embodiment of the invention, when the magazine is positioned to allow the insertion of one fastener into the patient, a portion of the magazine is accessible to allow the insertion of one or more additional fasteners into the magazine. In this fashion, additional fasteners may be added to the magazine without requiring its removal from the device or the removal of the conduit of the device from the patient.

In a preferred embodiment of the invention, the conduit or barrel of the device is easily removable from the rest of the device. This allows the same device to be used during an operation with differently shaped conduits, depending upon the location and condition of the tissue being treated. Thus, for instance, during the same operation, the surgeon could insert fasteners through a straight conduit, then easily replace the straight conduit with a curved conduit and continue the operation without the need for an entirely new device.

In another preferred embodiment of the invention, the pushing means, e.g., piston, of the device is capable of extending slightly beyond the end of the conduit that is in contact with the tissue being treated. Such extension of the pushing means ensures that it pushes the fastener until it is embedded entirely within the tissue being treated, with no portion of the fastener remaining on or above the surface of the tissue being treated.

In yet another preferred embodiment of this invention, the device has a safety mechanism that helps prevent the surgeon from inadvertently shooting the fastener into the patient until the proper moment. This mechanism works in conjunction with the triggering mechanism so that the means for propelling the pushing means rapidly into the fastener cannot be actuated until both the triggering means and the safety mechanism are actuated simultaneously.

### Brief Description of the Drawings

The invention will be more fully described in conjunction with the accompanying drawings wherein:
FIG. 1 is a side view, with parts broken away, of an embodiment of the device for installing tissue fasteners.
FIG. 2 is a perspective view of straight and curved conduits that may be used as part of the present invention.
FIG. 3 is a perspective view of various magazines for holding fasteners that may by used as part of the present invention.
FIG. 4 is a side view of the striking pin portion of the device when being cocked.
FIG. 5 is a side view of the striking pin, safety and triggering mechanisms that may be used as part of the present invention.
FIG. 6 is a side view of the striking pin that may be used as part of the present invention, as seen after inserting a fastener into a patient.
FIGS. 7 through 10 are side views, with parts broken away, of another embodiment of the device for installing tissue fasteners.

### Detailed Description of the Invention

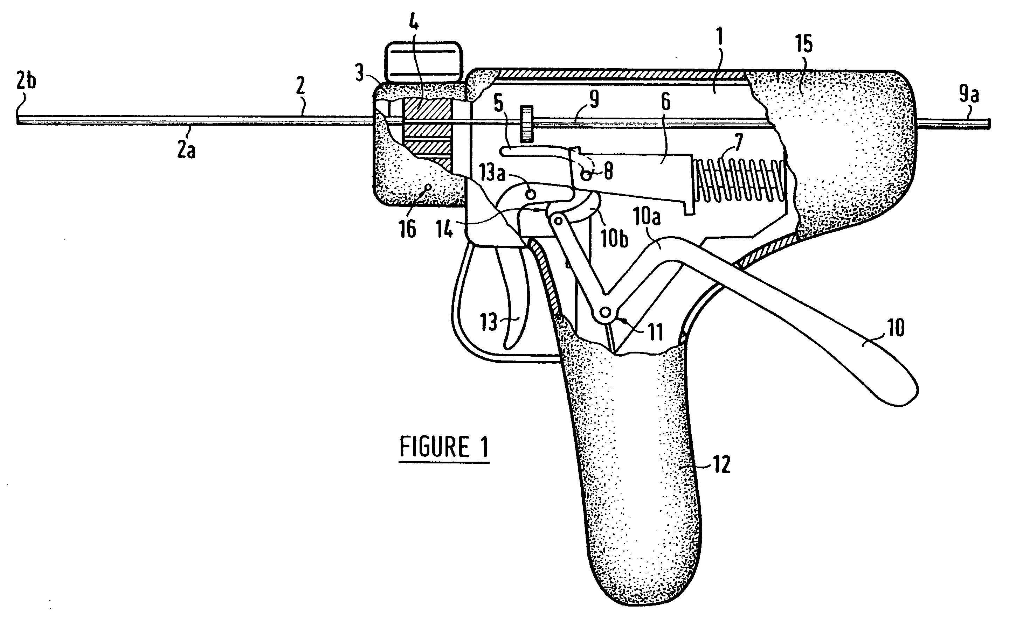

Referring to Figure 1, demonstrating a particular embodiment of the present invention, the present invention comprises a conduit 2, containing an internal channel, 2a, through which a fastener may be pushed. The conduit 2 terminates distally at end 2b, which is where the fastener exits the conduit 2 and is inserted into the tissue being treated. Thus, during the insertion procedure, the end 2b is placed in contact with the tissue to be treated. In a preferred embodiment of the invention, the end 2b is not perpendicular to the conduit 2, but rather is angled to provide better contact with the tissue to be treated. The end 2b can be smooth, or can be rough or contain points or other means that oppose the movement of the end 2b laterally across the surface of the tissue to be treated. Such points or other corresponding structures at end 2b stabilize the end 2b by preventing it from inadvertently slipping across the tissue being treated once the surgeon has properly positioned the end 2b of the conduit 2 in contact with the tissue where the fastener is to be inserted. In one embodiment of the present invention, the points or corresponding structure at the end 2b of the conduit 2 are retractable, thereby allowing easy movement of the end 2b of the conduit 2 within the patient when retracted, yet preventing slippage of the end 2b of the conduit 2 when extended. Thus, for instance, a surgeon could have the points retracted while positioning the end 2b of the conduit 2 within the patient, and then extend the points once he has properly positioned the end 2 of the conduit 2b within the patient and is ready to insert the fastener.

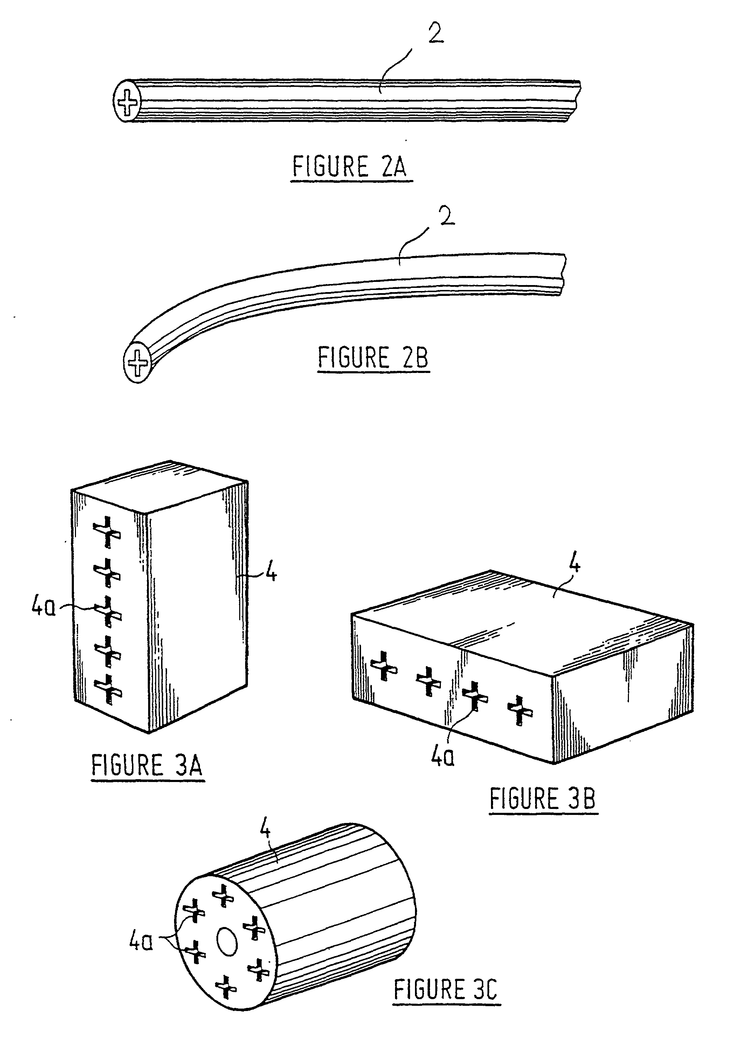

The geometry of the conduit 2 is variable, and will depend upon the type and location of the tissue being treated. Figure 2 demonstrates in side view two possible geometries for conduit 2. In a typical embodiment, the conduit 2 will have an elongated shape and a cross section that may vary depending upon the type and location of tissue to be treated. For instance, the conduit may have a circular or ellipsoidal cross section. The conduit 2 can be straight or contain gradual curves to allow easier, less invasive positioning of the end 2b within the patient.

The shape of the internal channel 2a is variable, and will depend upon the shape of the fastener being inserted. Preferably, the shape of the internal channel 2a is the same as the shape of the cross section of the fastener. This allows for accurate control of the angle and location of insertion of the fastener. The particular internal channels in Figure 2 are for use with fasteners having a cross-shaped cross section.

Referring again to Figure 1, in one preferred embodiment of the present invention, the conduit 2 is easily removable from the body 1 of the device 15. This enables a surgeon to conveniently replace a conduit during an operation in order to allow easier, quicker, less invasive positioning of fasteners. For instance, it may be advantageous to use a straight conduit for the insertion of some fasteners during an operation and advantageous to use a curved conduit for the insertion of other fasteners during the same operation. With the present invention, the surgeon can simply remove the straight conduit from the body of the device and replace it with a curved conduit and continue the operation. This affords the surgeon great flexibility and helps to insure that the surgery will be as quick and minimally invasive as possible, as well as cost efficient. The conduit 2 may be releasably attached to the body 1 at its proximal end by any of several methods that are well known for the releasable attachment of mechanical parts. For instance, the body 1 may contain a simple spring loaded release that, when compressed or pulled or otherwise manipulated, allows the removal and insertion of conduit 2, yet when left alone secures the conduit 2 within the body 1.

The proximal end of conduit 2 attaches to the body 1 near the fastener magazine seat 3. The magazine seat is capable of receiving a magazine 4 containing one or more fasteners. The magazine seat 3 is configured so that the magazine 4 may be easily positioned within the magazine seat 3 so that a fastener within the magazine 4 is aligned with the entrance to the internal channel 2a of the conduit 2. Once a fastener has been inserted into a patient, the magazine 4 may again be easily positioned within the magazine seat 3 so that another fastener contained within the magazine 4 is aligned with the entrance to the internal channel 2a of the conduit 2.

In a preferred embodiment of the invention, there is a magazine locking mechanism 16 that promotes alignment of the magazine 4 within the magazine seat 3 and ensures that, once aligned within the magazine seat 3, the magazine 4 does not inadvertently slip out of alignment. This magazine locking function can be achieved, for example, by a spring loaded ball bearing that slides against the side of the magazine 4 as it moves within the magazine seat 3. The side of the magazine 4 can contain slight indentations for receiving the spring loaded ball bearing when the magazine 4 is aligned. The presence of the spring loaded ball bearing in the indentation provides enough resistance to movement to prevent the magazine 4 from inadvertently slipping out of alignment, yet does not provide so much resistance that the surgeon cannot slide the magazine 4 within the magazine seat 3 to move another fastener into alignment. When the surgeon moves the magazine 4, the ball bearing slides out of the indentation and slides along the side of magazine 4 until it enters another indentation, signaling to the surgeon that the magazine is positioned so that another fastener is in proper alignment.

In one embodiment of the invention, after a fastener is inserted, the magazine 4 need not be manually positioned within the magazine seat 3 to align another fastener with the internal channel 2a of the conduit 2 because the magazine 4 contains means for automatically aligning another fastener once the previously-aligned fastener has been inserted. This means may be as simple as a spring contained within the magazine 4 that pushes the fasteners of the magazine sequentially into alignment for insertion.

The magazine 4 and magazine seat 3 may have a variety of geometries. Figure 3 demonstrates some potential geometries of magazines for use with this invention. It can be seen from Figures 3A and 3B that the magazine 4 may be box-shaped, with channels 4a for fasteners arranged linearly, either horizontally or vertically, within the magazine. Such box-shaped magazines may simply be pushed or pulled horizontally or vertically through or within the magazine seat in order to align a fastener for insertion. As shown in Figure 3C, in another embodiment of the invention, the magazine 4 may be cylindrically shaped, with the channels 4a for fasteners arranged circularly within the magazine 4. Such cylindrical magazines may simply be rotated within the magazine seat in order to align a fastener for insertion. The particular magazines of Figure 3 are for use with fasteners having a cross-shaped cross section, however the magazines may easily be configured to hold fasteners of various cross-sections.

Referring again to Figure 1, in one preferred embodiment of the present invention, when the magazine 4 is aligned within the magazine seat 3, a portion of the magazine 4 remains accessible to the surgeon or other medical personnel for the insertion of additional fasteners to the magazine 4. Thus, if the surgeon has just inserted the last fastener within a magazine into the patient, he can have another fastener inserted into the magazine, without even having to remove the magazine from the device. The magazine may then be positioned so that the newly-inserted fastener is aligned for insertion. By so positioning the magazine to align the fastener, another portion of the magazine will now be accessible to the surgeon for the insertion of another fastener. Thus, after inserting the aligned fastener into the patient, another fastener may be inserted into the magazine, without the magazine being removed from the magazine seat. The magazine may be positioned so that the newly-inserted fastener is aligned for insertion. Such positioning will make another portion of the magazine accessible to the surgeon for the insertion of another fastener into the magazine. In this fashion, the surgeon may continually replenish the magazine and thereby use an unlimited number of fasteners during an operation, without ever having to entirely remove the magazine from the device.

When a fastener within the magazine 4 is aligned with the internal channel 2a of conduit 2, it is also aligned with the means for pushing the fastener through the internal channel 2a and into the patient. In the embodiment shown in Figure 1, a piston 9 serves to push the fastener from the magazine 4, through the internal channel 2a, and into the patient. In the embodiment of Figure 1, the proximal end 9a of the piston 9 extends out from the body 1 of the device so that the piston 9 may be positioned manually. When the piston 9 is fully retracted, i.e., when the piston 9 is located as far away from the conduit 2 as possible, the distal end of the piston 9 is located proximally of the magazine seat 3, thereby allowing the magazine 4 to be positioned so that a fastener is aligned with the piston 9 and the internal channel 2a of the conduit 2. The piston 9 is capable of sliding through the magazine 4 in the magazine seat 3, thereby pushing the aligned fastener from the magazine 4 into the conduit 2. When fully extended, the piston 9 reaches the distal end 2b of the conduit 2 and is thereby capable of pushing the fastener from the conduit 2 into the patient. In a preferred embodiment of this invention, the piston 9 is capable of extending slightly beyond the distal end 2b of the conduit 2, thereby allowing it to push a fastener entirely within the tissue being treated, so that no part of the fastener remains above or on the tissue surface.

The piston 9 may be accelerated in several different ways. In Figure 1, the means for accelerating the piston 9 is a simple spring mechanism 7. Other possibilities include pneumatic, hydraulic, explosive, combustive, chemical or electromagnetic mechanisms. In Figure 1, the spring 7 is attached to a striking pin 6, which is capable of traveling along a loop 5 and thereby striking and accelerating the piston 9. In a preferred embodiment of this invention, there is a safety means 14 which prevents the inadvertent acceleration of the striking pin 6 into the piston 9.

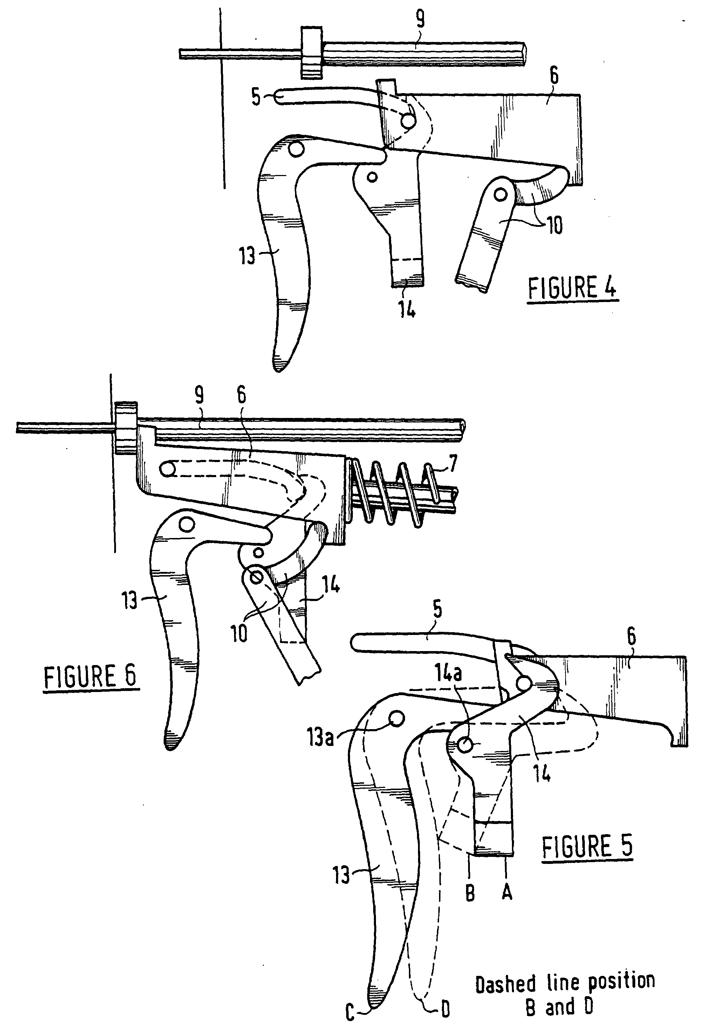

The operation of the particular embodiment of Figure 1 will now be described in detail. First; the spring mechanism 7 which is used to accelerate the piston 9 must be cocked. This is done by pushing the cocking lever 10 towards the handle 12 of the device. The cocking lever 10 pivots around point 10a, thereby causing the end 10b of the cocking lever to push the striking pin 6 in the proximal direction, thereby compressing the spring 7. The distal end of the striking pin 6 travels along the loop 5 in the proximal direction until it is pushed by the slanted end of the safety 14 into a notch 8 at the proximal end of the loop 5. When in the notch 8, the striking pin 6 cannot move distally. Thus, the spring 7 remains compressed. The safety 14 prevents the striking pin 6 from moving out of the notch 8, thereby preventing inadvertent release of the striking pin 6 and acceleration of the piston 9. Figure 4 shows the relative positions of the cocking lever 10, striking pin 6, piston 9, safety 14, and trigger 13 just after the striking pin has been cocked. As seen on Figure 1, the cocking lever 10 is returned to its previous position by a separate return spring 11.

Referring to Figures 1, 4 and 6, when the striking pin 6 is in the cocked position, the piston 9 may be freely positioned by the surgeon. The surgeon pulls the piston 9 proximally, so that the distal end of the piston 9 is located proximally of the magazine seat 3. A magazine 4 is inserted into the magazine seat 3 and positioned so that a fastener is aligned with the internal channel 2a of the conduit 2 and the piston 9. After positioning the device properly within the patient, so that the distal end 2b of the conduit 2 is in contact with the tissue to be treated, the surgeon may then push the piston 9 distally, so that the distal end of the piston 9 travels through the magazine 4, thereby pushing the fastener into internal channel 2a of the conduit 2. The piston 9 may be pushed until the fastener reaches the distal end 2b of the conduit 2 and contacts the tissue to be treated.

Referring to Figures 1 and 5, the surgeon must then release the safety 14 by pushing the safety lever 14 distally. When the safety 14 is in position A, the top end of the safety 14 holds the striking pin 6 in place in the notch 8 on the loop 5. When the safety lever 14 is moved distally to position B, the lever 14 pivots around point 14a, and the top of the safety lever 14 moves proximally and downward, thereby no longer preventing the striking pin 6 from moving distally and upward when moved out of the notch 8 on the loop 5 by the triggering means 13. While holding the safety lever in position B, the surgeon then pulls the trigger 13 proximally, from position C to position D as shown in Figure 5. This causes the end of the trigger 13, which pivots around point 13a, to push the striking pin 6 off of the notch 8 on the loop 5. The striking pin 6, now removed from the notch 8, is accelerated rapidly along the loop 5 in the distal direction by the compressed spring 7. As it travels along the loop 5, the striking pin 6 strikes the piston 9, thereby accelerating it rapidly in the distal direction. The distal end of the piston 9 pushes the fastener into the tissue to be treated. The distal movement of the piston 9 stops once the distal end of the piston is at, or in a preferred embodiment of the invention, slightly beyond, the distal end 2b of the conduit 2. Figure 6 shows the relative positions of the cocking lever 10, striking pin 6, piston 9, safety 14, and trigger 13 after the striking pin 6 has accelerated the piston 9 so that it has inserted a fastener into the patient.

In order to insert another fastener, the surgeon then merely repeats the above process, except that he need not insert another magazine, but rather merely reposition the magazine that has already been inserted into the device so that another fastener is aligned with the internal channel 2a of the conduit 2.

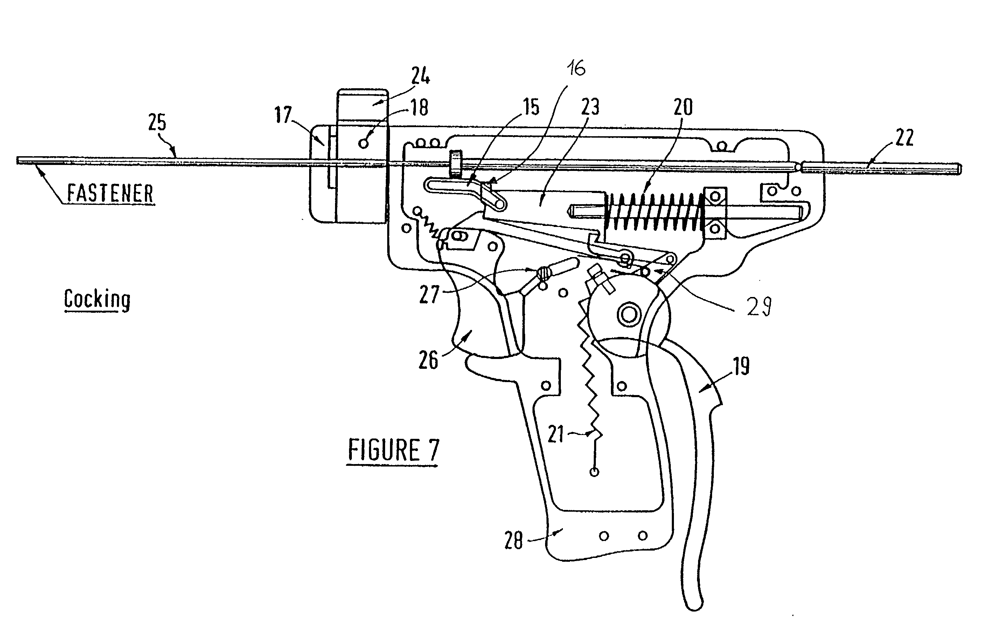

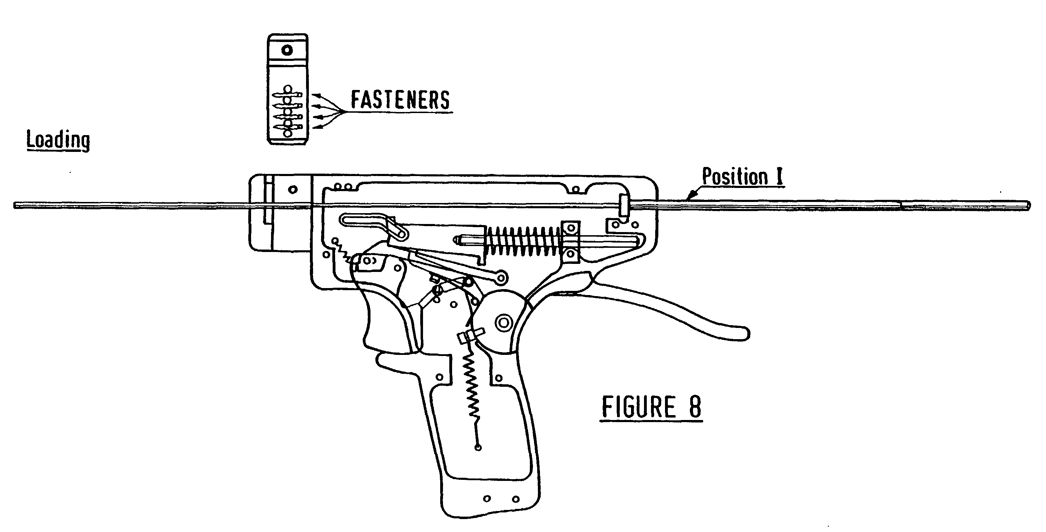

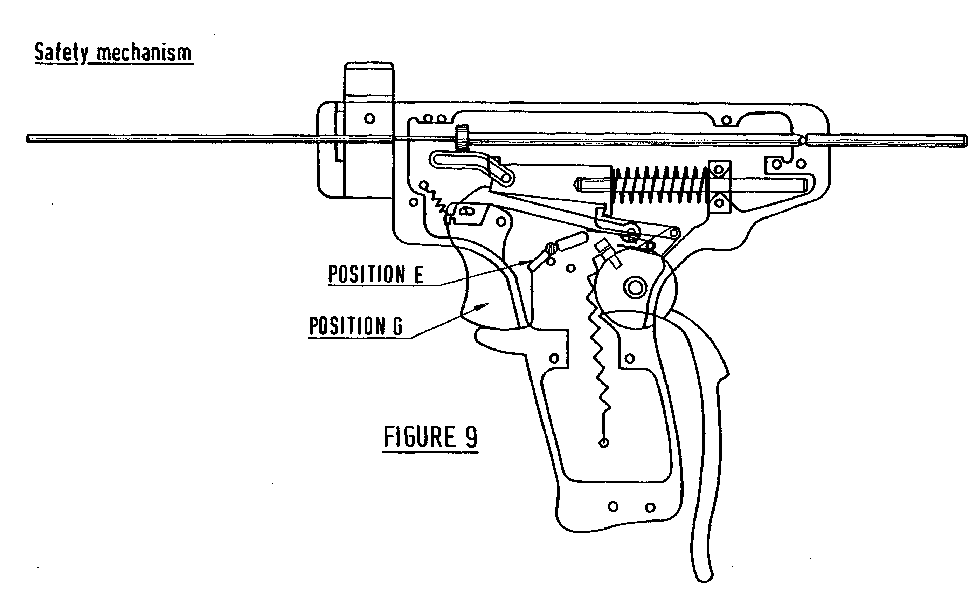

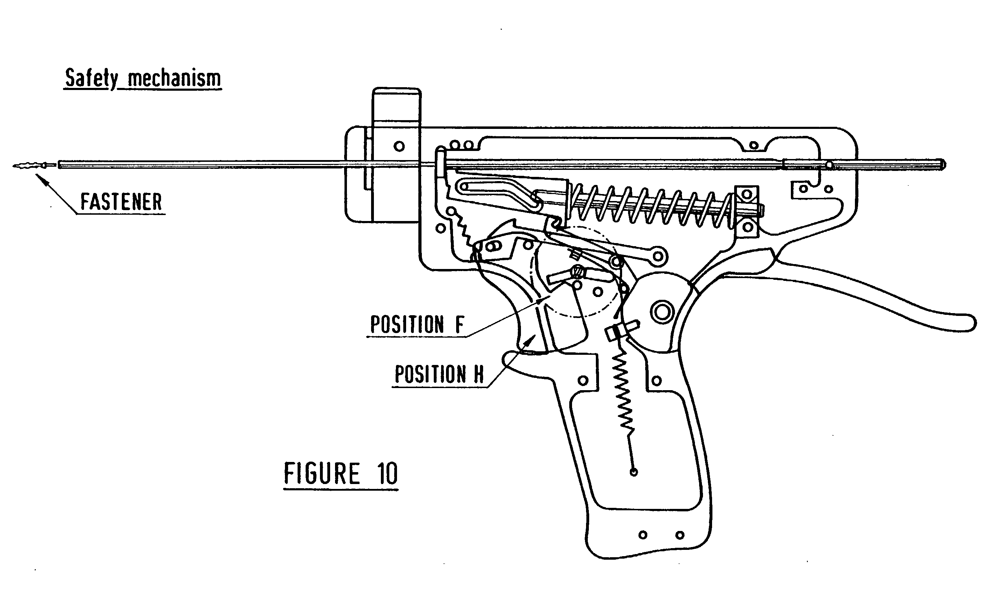

Figures 7 through 10 depict another preferred embodiment of the invention and its method of operation. Referring to those Figures (7-10), the device is cocked by pressing the cocking lever 19 forward against the hand grip 28, so that the cocking mechanism 29 cocks the striking pin 23, by pushing the striking pin 23 against the spring 20 and compressing it. After cocking, the safety mechanism 27 automatically locks the trigger 26 in place and a separate return spring 21 returns the cocking lever 19 to its original position (as in Figure 8). The device is loaded by manually pulling the piston 22 back to the I position (*see* Figure 8), placing a cartridge magazine 24 in the cartridge magazine seat 17, and fastening that magazine in place with the locking mechanism 18. The piston 22 is then pushed forward so the tip of the piston passes through the cartridge magazine, thereby moving a fastener into the canule tube 25, about 20 mm from the tip of the canule. The tip of the canule 25 is placed firmly against the torn meniscus so that the edges of the meniscus are pressed against each other, the safety mechanism of the device is released by pushing the safety lever 27 from position E to position F (see Figures 9 and 10), and the device is fired by pulling the trigger 26 from position G to position H (*see* Figures 9 and 10). The device can be fired only if the safety lever is in position F. Pulling the trigger 26 in this manner releases the striking pin 23, which is then moved forward by the energy of the spring 20, as it decompresses. The striking pin 23 moves forward in the loop 15, which also moves the piston 22 forward by way of a notch 16. The tip of the piston 22 then pushes the fastener into the meniscus, while the tip of the piston stops at the end of the canule 25. The entire procedure can be repeated by cocking the spring 20 with the cocking lever 19, pulling the piston 22 back, and pressing the cartridge magazine 24 down, so the next chamber containing a fastener lines up with the canule 25.

After the description above of the present invention and certain specific embodiments thereof, it will be readily apparent to those skilled in the art that many variations and modifications may be made to the present invention without departing from the scope of the appended claims.

## Claims

1. A device for installing tissue fasteners in a patient, comprising:
a conduit (2) for delivering a fastener into the patient, said conduit (2) having an interior shape and a distal end (2b) for insertion into the patient,
a tissue fastener magazine (4), said magazine being capable of removably receiving one or more tissue fasteners,
a body (1) having a seat (3) capable of removably receiving said tissue fastener magazine (4), so that each of said fasteners is capable of being aligned with said conduit (2) while said distal end (2b) of said conduit is located within said patient, and
means for moving said tissue fasteners from said seat (3) into said conduit (2) and the patient,
**characterized by** said magazine (4) being capable of receiving at least one fastener while said magazine (4) is located at least partially within said seat (3) and while said distal end (2b) of said conduit (2) is located within said patient.

2. The device of claim 1, further comprising a trigger (26) for causing said means for moving said tissue fasteners from said conduit (2) into the patient when the trigger (26) is moved from a first position to a second position, and cocking means for moving said trigger (26) from the second position to the first position when the cocking means is moved from an uncocked position to a cocked position.

3. The device of claim 2, further comprising spring means (20) connected to said cocking means for automatically moving said cocking means from the cocked position to the uncocked position when the trigger (26) is moved from the first position to the second position.

4. The device of any of the preceding claims, wherein said tissue fastener magazine (4) is capable of being removed from said seat (3) while said conduit is located within said patient.

5. The device of any of the preceding claims, wherein said means for moving said tissue fasteners comprises a piston (9), and wherein said piston (9) is capable of sliding through said conduit (2) and extending beyond said distal end (2b) of said conduit (2).

6. The device of any of the preceding claims, wherein said conduit (2) is removably attached to said body (1).

7. The device of any of the preceding claims, further comprising a retractable stabilizer located on said distal end (2b) of said conduit (2).

8. The device of any of the preceding claims, wherein said interior shape of said conduit (2) is the same as the greatest cross section of said tissue fasteners.

9. The device of any of the preceding claims, wherein the means for moving said tissue fasteners is capable of moving fasteners at more than one speed.

10. The device of claim 9, wherein the means for moving said tissue fasteners is capable of moving said tissue fasteners from said magazine (4) to said distal end (2b) of said conduit (2) at a rate that is slower than the rate at which the means for moving said tissue fasteners moves said tissue fasteners from said distal end (2b) of said conduit (2) into said patient.

## Patentansprüche

1. Vorrichtung zum Anbringen von Gewebebefestigungselementen in einem Patienten mit:
einem Kanal (2) zum Einbringen eines Befestigungselementes in den Patienten, wobei der Kanal (2) eine innere Form hat und ein distales Ende (2b) zum Einsetzen in den Patienten,
einem Gewebebefestigungselement-Magazin (4), wobei das Magazin ein oder mehr Gewebebefestigungselemente entfernbar aufnehmen kann,
einem Körper (1) mit einem Sitz (3), der das Gewebebefestigungselement-Magazin (4) entfernbar aufnehmen kann, so dass jedes der Befestigungselemente zu dem Kanal (2) ausgerichtet werden kann, während das distale Ende (2b) des Kanals in dem Patienten angeordnet ist, und
Mitteln zum Bewegen des Gewebebefestigungselementes von dem Sitz (3) in den Kanal (2) und den Patienten,
**dadurch gekennzeichnet, dass** das Magazin (4) in der Lage ist, wenigstens ein Befestigungselement aufzunehmen, während das Magazin (4) wenigstens teilweise innerhalb des Sitzes (3) angeordnet ist und während das distale Ende (2b) des Kanals (2) innerhalb des Patienten angeordnet ist.

2. Vorrichtung nach Anspruch 1, außerdem mit einem Auslöser (26) um zu bewirken, dass die Mittel zum Bewegen des Gewebebefestigungselementes von dem Kanal (2) in den Patienten, wenn der Auslöser (26) von einer ersten Position zu einer zweiten Position bewegt wird, und einem Spannmittel zum Bewegen des Auslösers (26) von der zweiten Position zu der ersten Position, wenn das Spannmittel von einer ungespannten Position zu einer gespannten Position bewegt wird.

3. Vorrichtung nach Anspruch 2, außerdem mit Federmitteln (20), die mit den Spannmitteln verbunden sind, um die Spannmittel automatisch von der gespannten Position zu der ungespannten Position zu bewegen, wenn der Auslöser (26) von der ersten Position zu der zweiten Position bewegt wird.

4. Vorrichtung nach einem der vorhergehenden Ansprüche, wobei das Gewebebefestigungselement-Magazin (4) in der Lage ist, von dem Sitz (3) entfernt zu werden, während der Kanal innerhalb des Patienten angeordnet ist.

5. Vorrichtung nach einem der vorhergehenden Ansprüche, wobei die Mittel zum Bewegen der Gewebebefestigungselemente einen Kolben (9) aufweisen, und wobei der Kolben (9) in der Lage ist, durch den Kanal (2) zu gleiten und sich über das distale Ende (2b) des Kolbens (2) zu erstrecken.

6. Vorrichtung nach einem der vorhergehenden Ansprüche, wobei der Kanal (2) entfernbar an dem Körper (1) befestigt ist.

7. Vorrichtung nach einem der vorhergehenden Ansprüche, außerdem mit einem rückziehbaren Stabilisator, der an dem distalen Ende (2b) des Kanals (2) angeordnet ist.

8. Vorrichtung nach einem der vorhergehenden Ansprüche, wobei die innere Form des Kanals (2) die gleiche ist wie der größte Querschnitt der Gewebebefestigungselemente.

9. Vorrichtung nach einem der vorhergehenden Ansprüche, wobei die Mittel zum Bewegen der Gewebebefestigungselemente in der Lage sind, Befestigungselemente bei mehr als einer Geschwindigkeit zu bewegen.

10. Vorrichtung nach Anspruch 9, wobei die Mittel zum Bewegen der Gewebebefestigungselemente in der Lage sind, die Gewebebefestigungselemente von dem Magazin (4) zu dem distalen Ende (2b) des Kanals (2) mit einer Rate zu bewegen, die langsamer ist als die Rate, mit der die Mittel zum Bewegen der Gewebebefestigungselemente die Gewebebefestigungselemente von dem distalen Ende (2b) des Kanals (2) in den Patienten bewegen.

## Revendications

1. Dispositif pour placer un élément de fixation de tissu dans un patient, comprenant :
- un conduit (2) pour délivrer un élément de fixation dans un patient, ledit conduit (2) ayant une forme intérieure et une extrémité distale (2b) destinée à être introduite dans le patient,
- un magasin pour élément de fixation de tissu (4), ledit magasin étant apte à recevoir de façon amovible un ou plusieurs éléments de fixation de tissu,
- un corps (1) ayant un siège (3) apte à recevoir de façon amovible ledit magasin pour éléments de fixation de tissu (4) de telle sorte que chacun desdits éléments de fixation est apte à être aligné avec ledit conduit (2) lorsque ladite extrémité distale (2b) dudit conduit est située à l'intérieur dudit patient, et
- des moyens pour déplacer ledit élément de fixation de tissu dudit siège (3) dans ledit conduit (2) et le patient,
**caractérisé en ce que** ledit magasin (4) est apte à recevoir au moins un élément de fixation lorsque ledit magasin (4) est situé au moins partiellement à l'intérieur dudit siège (3) et lorsque ladite extrémité distale (2b) dudit conduit (2) est située à l'intérieur du patient.

2. Dispositif selon la revendication 1, comprenant en outre une gâchette (26) pour déclencher lesdits moyens pour déplacer ledit élément de fixation de tissu dudit siège (3) dans le patient lorsque la gâchette (26) est déplacée d'une première position dans une deuxième position, et des moyens pour armer pour déplacer ladite gâchette (26) de la seconde position vers la première position lorsque les moyens pour armer sont déplacés d'une position non armée vers une position armée.

3. Dispositif selon la revendication 2, comprenant en outre des moyens ressort (20) connectés aux dits moyens pour armer pour déplacer automatiquement lesdits moyens pour armer de la position armée vers la position non armée lorsque la gâchette (26) est déplacée de la première position dans la deuxième position.

4. Dispositif selon l'une des revendications précédentes, dans lequel le magasin pour éléments de fixation est apte à être enlevé dudit siège (3) lorsque ledit conduit (2) est situé à l'intérieur du patient.

5. Dispositif selon l'une des revendications précédentes, dans lequel lesdits moyens pour déplacer les éléments de fixation du tissu comprennent un piston (9), et dans lequel ledit piston (9) est apte à glisser à travers le conduit (2) et à s'étendre au-delà de ladite extrémité distale (2b) dudit conduit (2).

6. Dispositif selon l'une des revendications précédentes, dans lequel ledit conduit (2) est attaché de façon amovible audit corps (1).

7. Dispositif selon l'une des revendications précédentes, comprenant en outre un stabilisateur rétractable situé sur ladite extrémité distale (2b) dudit conduit (2).

8. Dispositif selon l'une des revendications précédentes, dans lequel ladite forme intérieure dudit conduit (2) est la même que la plus grande section transversale desdits éléments de fixation de tissu.

9. Dispositif selon l'une des revendications précédentes, dans lequel les moyens pour déplacer les éléments de fixation de tissu sont aptes à déplacer les éléments de fixation à plus d'une vitesse.

10. Dispositif selon la revendication 9, dans lequel les moyens pour déplacer lesdits éléments de fixation de tissu sont aptes à déplacer lesdits éléments de fixation de tissu dudit magasin (4) dans ladite extrémité distale (2b) dudit conduit (2) à une vitesse qui est plus lente que la vitesse à laquelle les moyens pour déplacer lesdits éléments de fixation de tissu déplacent lesdits éléments de fixation de tissu de ladite extrémité distale (2b) dudit conduit (2) à l'intérieur du patient.
